# EUROPEAN PATENT APPLICATION

(11) **EP 4 509 524 A1**
(43) Date of publication of application: **19.02.2025**
(21) Application number: 23787804.6
(22) Date of filing: 13.04.2023
(51) Int. Cl.: C07K 14/735, C07K 19/00, C12N 15/12, C12N 15/62, C12N 15/63, C12N 5/10, A61K 38/17, A61K 35/17, A61P 35/00

(54) **ANTI-SPLICING MUTANT OF CD16 FOR ENHANCING CELL FUNCTION**

(30) Priority: 13.04.2022 CN 202210398177
(71) Applicant: Neukio Biotherapeutics (Shangai ) Co., Ltd., Shanghai 200131 (CN)
(72) Inventor: ZHANG, Lei, Shanghai 200131 (CN); WANG, Yuan, Shanghai 200131 (CN); ZU, Rui, Shanghai 200131 (CN)
(74) Representative: Mewburn Ellis LLP
(86) International application number: PCT/CN2023/088179
(87) International publication number: WO 2023/198162

(57) **Abstract**

Provided in the present invention is an anti-cleavage mutant of CD16 for enhancing cell function. Specifically, the CD16 mutant of the present invention has an amino acid residue mutation at the following sites corresponding to a wild-type CD16: insertion of one or more proline between V196 and S197 sites and/or between S197 and T198 sites of CD16-F176V. The CD16 mutant of the present invention can resist the cleavage of ADAM17, and can enhance the ADCC capability of NK92 cell line expressing same.

## Description

### Technical field

The present invention relates to the field of immunology. Specifically, it relates to an anti-cleavage mutant of CD16 for enhancing cell function.

### Background

FcγRIII (CD16) is a glycoprotein with a molecular weight ranging from 50 to 70 kDa, belonging to the Ig superfamily. It possesses two C2 domains and its gene is located on chromosome 1q23~24. FcγRIII binds to human IgG and IgG3, serving as a low-affinity receptor for antibodies. In the general population, wild-type CD16 exhibits polymorphism, and variations at the F176V site can enhance its affinity for binding to IgG and IgG3.

ADCC is a process involving the activation of NK cells, the release of cytokines and cytolytic granules, as well as the induction of apoptosis in target cells. It is primarily induced and produced by the binding of low-affinity-mediated Fc receptor CD16 (FcγRIIIA or CD16a) and antibodies. Direct activation of NK cells through CD16 promotes the formation of immune synapses between NK cells and tumor cells, demonstrating cytotoxic activity against various tumor cells.

During this process, CD16 acts as the receptor that activates NK cell effector functions, inducing phosphorylation of immunoreceptor tyrosine-based activation motifs (ITAMs) through signal transduction, triggering the release of lytic granules and cytokines such as IFN-γ and TNF-α.

Moreover, when NK cells interact with target cells, CD16 on NK cells undergoes downregulation of membrane expression levels while transmitting activation signals. This is a result of shedding of the extracellular fragment of CD16 during NK cell activation. Shedding of CD16 from activated NK cells is mediated by metalloproteinase 17 (ADAM17). This demonstrates the central importance of CD16 in triggering NK cell-mediated ADCC.

Considering that CD16 shedding negatively regulates the ADCC function of cells expressing it, such as NK cells, and weakens the efficacy of antibody-based therapies over time, there is a need in the field to develop engineered CD16 mutants that are resistant to cleavage by ADAM17 and enhance the ADCC capability of NK cells, in order to overcome the effects of this negative regulatory signal.

### Summary of the invention

The purpose of the present invention is to provide an engineered CD16 mutant.

The purpose of the present invention further lies in providing an anti-cleavage mutant of CD16 for enhancing cell function.

In the first aspect of the present invention, it provides a CD16 mutant, wherein the CD16 mutant undergoes amino acid residue mutations at the following sites corresponding to wild-type CD16: insertion of one or more amino acids between the V196 and S197 sites and/or between the S197 and T198 sites of wild-type CD16; wherein the insertion of one or more amino acids comprises the insertion of one or more prolines.

In another preferred embodiment, the CD16 mutant further comprises a mutation where the F at position 176 of wild-type CD16 is replaced with V (F176V).

In another preferred embodiment, the mutation further comprises the deletion of one or more amino acids, or the substitution of one or more amino acids.

In another preferred embodiment, the insertion of one or more amino acids is the insertion of 1, 2, or 3 prolines.

In another preferred embodiment, the insertion of one or more amino acids comprises:
(1) insertion of 1 or 2 P between the V196 and S197 sites of wild-type CD16;
(2) insertion of 1 or 2 P between the S197 and T198 sites of wild-type CD16; or
(3) a combination of any of the above.

In another preferred embodiment, the insertion of one or more amino acids comprises:
(1) insertion of 1 P between the V196 and S197 sites of wild-type CD16;
(2) insertion of 1 P between the S197 and T198 sites of wild-type CD16; or
(3) insertion of 1 P between the V196 and S197 sites of wild-type CD16, and insertion of 1 P between the S197 and T198 sites of wild-type CD16.

In another preferred embodiment, the CD16 mutant further comprises a mutation where the F at position 176 of wild-type CD16 is replaced with V (F176V).

In another preferred embodiment, the insertion of one or more amino acids comprises:
(1) insertion of 1 or 2 P between the V196 and S197 sites of CD16-F176V;
(2) insertion of 1 or 2 P between the S197 and T198 sites of CD16-F176V; or
(3) a combination of any of the above.

In another preferred embodiment, the insertion of one or more amino acids comprises:
(1) insertion of 1 P between the V196 and S197 sites of CD16-F176V;
(2) insertion of 1 P between the S197 and T198 sites of CD16-F176V; or
(3) insertion of 1 P between the V196 and S197 sites of CD16-F176V, and insertion of 1 P between the S197 and T198 sites of CD16-F176V.

In another preferred embodiment, the CD16 mutant is selected from the group consisting of:
(1) CD16-F176V-V196_S197insP, with the amino acid sequence as shown in SEQ ID NO. 3,
(2) CD16-F176V-S197_T198insP, with the amino acid sequence as shown in SEQ ID NO. 4, and
(3) CD16-F176V-V196_S197insP-V197_T198insP, with the amino acid sequence as shown in SEQ ID NO. 5.

In another preferred embodiment, the amino acid sequence of the wild-type CD16 is as shown in SEQ ID NO. 1.

In another preferred embodiment, the amino acid sequence of CD16-F176V is as shown in SEQ ID NO. 6. In another preferred embodiment, compared to the wild-type CD16 polypeptide, the CD16 mutant exhibits reduced sensitivity to ADAM17-mediated shedding.

In another preferred embodiment, compared to the wild-type CD16 polypeptide, the CD16 mutant is resistant to cleavage by ADAM17.

In the second aspect of the present invention, it provides a fusion protein, which comprises the CD16 mutant of the first aspect of the present invention and a functional portion that is not a CD16 mutant.

In another preferred embodiment, the functional portion that is not a CD16 is selected from the group consisting of:
Fc fragments, including but not limited to: Fc fragments of human IgG1, IgG2, IgG3, or IgG4, and Fc fragment mutants thereof with homology of 90% or higher;
human serum albumin (HSA);
6His tag.

In a preferred embodiment, the CD16 mutant and the functional portion that is not a CD16 in the fusion protein can be directly linked or linked via a linker; the linker may be a repeated sequence of AAA or GS, including but not limited to a repeated sequences of G3S or G4S; for example, (G3S)4.

In the third aspect of the present invention, it provides a polynucleotide, which encodes the CD16 mutant of the first aspect of the present invention or the fusion protein of the second aspect of the present invention.

In the fourth aspect of the present invention, it provides an expression vector, which comprises the polynucleotide of the third aspect of the present invention.

In the fifth aspect of the present invention, it provides a cell, which comprises the expression vector of the fourth aspect of the present invention, or the genome of the cell is integrated with the polynucleotide of the third aspect of the present invention.

In another preferred embodiment, the cell expresses the CD16 mutant as described above.

In another preferred embodiment, the cell is a eukaryotic cell; preferably a yeast, an insect cell, or an animal cell; it may be a mammalian cell.

In another preferred embodiment, the cell is a natural killer (NK) cell, neutrophil, monocyte, T cell, macrophage, pluripotent stem cell, or a differentiated cell derived from the pluripotent stem cell.

In another preferred embodiment, the pluripotent stem cell comprises a human induced pluripotent stem cell (iPSC) or human embryonic stem cell (ESC), or the differentiated cell derived from the pluripotent stem cell is a hematopoietic cell.

In another preferred embodiment, the NK cell is a NK cell derived from the human induced pluripotent stem cell (iPSC) and human embryonic stem cell (ESC), or a NK cell induced or expanded from peripheral blood or umbilical cord blood.

In another preferred embodiment, the cell is a natural killer (NK) cell, and compared to NK cells expressing a CD16 polypeptide comprising the amino acid sequence as shown in SEQ ID NO. 1, SEQ ID NO. 6, or SEQ ID NO. 2, the NK cell exhibits one or more characteristics selected from the following:
(a) increased antitumor capacity;
(b) increased antiviral capacity;
(c) enhanced antibody-dependent cellular cytotoxicity (ADCC);
(d) increased production of IFNγ or TNFα;
(e) increased CD16-mediated activity;
(f) higher CD16 surface levels;
(g) enhanced cellular stimulation; and
(h) increased anticancer activity *in vivo.*

In another preferred embodiment, compared to NK cells expressing a CD16 polypeptide comprising the amino acid sequence as shown in SEQ ID NO. 1, SEQ ID NO. 6, or SEQ ID NO. 2, the ADCC capacity of the NK cell is increased by at least ≥20%, ≥30%, ≥40%, ≥50%, or higher, such as 20-90% or 30-80%.

In the sixth aspect of the present invention, it provides a pharmaceutical composition, which comprises the CD16 mutant of the first aspect of the present invention, the fusion protein of the second aspect of the present invention, or the cell of the fifth aspect of the present invention, and a pharmaceutically acceptable carrier.

In another preferred embodiment, the pharmaceutical composition is a formulation.

In another preferred embodiment, the pharmaceutical composition is used for treating tumors or enhancing ADCC activity in mammalian cells.

In another preferred embodiment, the pharmaceutical composition further comprises other drugs used for treating tumors.

In another preferred embodiment, the other drugs used for treating tumors (i) specifically recognize tumor antigens; or (ii) comprise antibodies or antibody fragments that specifically recognize tumor antigens; or (iii) specifically recognize viral target proteins.

In another preferred embodiment, the other drugs used for treating tumors comprise therapeutic NK cell drugs.

In another preferred embodiment, the tumor antigens comprise HER2, CD20, EGFR, CD38, CD3, CD4, CD8, CD19, CD22, CD34, CD40, CD52, HER1, HER3, HER4, VCAM, CD11a, CD18, CD11b, VEGF, and Claudin18.2; or the antibodies comprise trastuzumab, rituximab, pertuzumab, ranibizumab, ublituximab, omalizumab, isatuximab, ofatumumab, inebilizumab, cetuximab, panitumumab, nimotuzumab, obinutuzumab, alemtuzumab, futuximab, imgatuzumab, matuzumab, and ertumaxomab.

In another preferred embodiment, the antibodies or antibody fragments that specifically recognize tumor antigens are antibodies with significant ADCC activity; preferably trastuzumab, cetuximab, rituximab, pertuzumab, ranibizumab, ublituximab, omalizumab, isatuximab, ofatumumab, inebilizumab, panitumumab, nimotuzumab, obinutuzumab, alemtuzumab, futuximab, imgatuzumab, matuzumab, or ertumaxomab; and more preferably trastuzumab, cetuximab, or rituximab.

In the seventh aspect of the present invention, it provides an *in vitro* method for enhancing ADCC activity in mammalian cells, which comprises introducing the polynucleotide of the third aspect of the present invention into the cells.

In the eighth aspect of the present invention, it provides a use of the CD16 mutant of the first aspect of the present invention, the fusion protein of the second aspect of the present invention, or the cell of the fifth aspect of the present invention in the manufacture of a medicament for expanding T lymphocytes or natural killer (NK) cells *in vitro* or for treating a disease in an individual.

In another preferred embodiment, the disease is a disease treated with NK cells as immunotherapy.

In another preferred embodiment, the disease is a disease treated by stimulating the immune system or by proliferating immune cells, for example, stimulating the immune system to mediate ADCC to kill tumor cells.

In another preferred embodiment, the disease is cancer or a tumor, such as lymphoma, colorectal cancer, breast cancer, lung cancer, gastric cancer, liver cancer, multiple myeloma, renal cancer, pancreatic cancer, melanoma, thyroid cancer, brain tumors, skin cancer, prostate cancer, ovarian cancer, cervical cancer, head and neck cancer, bladder cancer, or a combination thereof.

In the ninth aspect of the present invention, it provides a method for treating tumors, which involves administering the CD16 mutant of the first aspect of the present invention, the fusion protein of the second aspect of the present invention, the cell of the fifth aspect of the present invention, or the pharmaceutical composition of the sixth aspect of the present invention, to a subject in need thereof.

In another preferred embodiment, the method further comprises administering a therapeutic NK cell drug to the subject.

In another preferred embodiment, the NK cell drug (i) specifically recognizes tumor antigens; or (ii) comprises antibodies or antibody fragments that specifically recognize tumor antigens; or (iii) specifically recognizes viral target proteins.

In another preferred embodiment, the tumor antigens comprise HER2, CD20, EGFR, CD38, CD3, CD4, CD8, CD19, CD22, CD34, CD40, CD52, HER1, HER3, HER4, VCAM, CD11a, CD18, CD11b, VEGF, and Claudin18.2; or the antibodies comprise trastuzumab, rituximab, pertuzumab, ranibizumab, ublituximab, omalizumab, isatuximab, ofatumumab, inebilizumab, cetuximab, panitumumab, nimotuzumab, obinutuzumab, alemtuzumab, futuximab, imgatuzumab, matuzumab, and ertumaxomab.

In another preferred embodiment, the antibodies or antibody fragments that specifically recognize tumor antigens are antibodies with significant ADCC activity.

In the tenth aspect of the present invention, it provides an active ingredient combination, which comprises a first active ingredient: the CD16 mutant of the first aspect of the present invention, the fusion protein of the second aspect of the present invention, or the cell of the fifth aspect of the present invention; and a second active ingredient: an antibody or antibody fragment that specifically recognizes tumor antigens.

In another preferred embodiment, the active ingredient combination is used for enhancing ADCC activity in mammalian cells.

In another preferred embodiment, the antibody or antibody fragment that specifically recognizes tumor antigens is an antibody with significant ADCC activity.

In the eleventh aspect of the present invention, it provides a use of an active ingredient combination, wherein the active ingredient combination comprises a first active ingredient: the CD16 mutant of the first aspect of the present invention, the fusion protein of the second aspect of the present invention, or the cell of the fifth aspect of the present invention;
and a second active ingredient: an antibody or antibody fragment that specifically recognizes tumor antigens, and the combination is used for: enhancing ADCC activity in mammalian cells; and/or preparing a pharmaceutical composition or kit for treating tumors.

In another preferred embodiment, the combination is used for preparing a pharmaceutical composition or kit for synergistic treatment of tumors.

In another preferred embodiment, the antibody or antibody fragment that specifically recognizes tumor antigens is an antibody with significant ADCC activity; preferably trastuzumab, cetuximab, rituximab, pertuzumab, ranibizumab, ublituximab, omalizumab, isatuximab, ofatumumab, inebilizumab, panitumumab, nimotuzumab, obinutuzumab, alemtuzumab, futuximab, imgatuzumab, matuzumab, or ertumaxomab; and more preferably trastuzumab, cetuximab, or rituximab.

In another preferred embodiment, the tumor antigens comprise HER2, CD20, EGFR, CD38, CD3, CD4, CD8, CD19, CD22, CD34, CD40, CD52, HER1, HER3, HER4, VCAM, CD11a, CD18, CD11b, VEGF, and Claudin18.2.

It should be understood that within the scope of the present invention, the above-mentioned technical features of the present invention and the technical features specifically described in the following (such as the examples) can be combined with each other to form a new or preferred technical solution, which is not redundantly repeated one by one herein due to space limitation.

### Description of the drawings

Figure 1 illustrates the schematic diagram of cleavage of CD16 by ADAM17.
Figure 2 illustrates the X-ray model of the ADAM17 protein.
Figure 3 illustrates that the CD16 mutant of the present invention can resist cleavage by ADAM17.
Figure 4 illustrates that the CD16 mutant of the present invention mediates stronger NK92 cell activation.
Figures 5A and 5B illustrate that the CD16 mutant of the present invention mediates stronger ADCC effects.
Figure 6 illustrates that iNKs expressing CD16-mut3 exhibit resistance to cleavage (Figure 6A) and stronger ADCC capability (Figure 6B).
Figure 7A illustrates strong ADCC effects of iNKs expressing CD16-mut3 on A549 tumor cells;
Figure 7B illustrates strong ADCC effects of iNKs expressing CD16-mut3 on Cal27 tumor cells;
Figure 7C illustrates strong ADCC effects of iNKs expressing CD16-mut3 on Cal33 tumor cells;
Figure 7D illustrates strong ADCC effects of iNKs expressing CD16-mut3 on FADU tumor cells;
Figure 7E illustrates strong ADCC effects of iNKs expressing CD16-mut3 on SKOV3 tumor cells.

### Detailed description

After extensive and in-depth research, the inventors of the present invention unexpectedly discovered for the first time that three conformational mutants of CD16, designed based on the binding mode between CD16 and the enzymatic active pocket region of ADAM17, can resist cleavage by ADAM17 and enhance the ADCC capacity of NK cells or NK92 cell lines that express them. They further avoid the shedding of CD16 from NK cells, thereby enhancing NK cell stimulation and cancer cell killing. On this basis, the present invention has been completed.

### Terms

### FcγRIII and NK cells

FcγRIII (CD16) is a glycoprotein with a molecular weight ranging from 50 to 70 kDa, belonging to the Ig superfamily and possessing two C2 structures. Its gene is located on chromosome 1q23~24. FcγRIII binds to human IgG and IgG3, serving as a low-affinity receptor for antibodies.

There are two isoforms of FcγRIII: FcγRIIIA and FcγRIIIB. (1) FcγRIIIA, it has a transmembrane structure and is mainly distributed in macrophages, NK cells, and eosinophils. Macrophages express high levels of FcγRIIIA, while monocytes express it at lower levels. FcγRIIIA is associated with CD3ζ or FcεRIγ chain dimer linked by disulfide bonds, FcγRIIIA on macrophages is associated with the CD3 complex γ chain, and FcγRIIIA on NK/LGL is associated with the ζ chain. TGF-β promotes the expression of FcγRIIIA in cultured monocytes. (2) FcγRIIIB, it is "anchored" to the surface of neutrophils via a GPI, and is expressed on each human neutrophil at levels ranging from 100,000 to 200,000 copies. The majority of soluble FcγRIII in the blood originates from this form. Short-term treatment with neutrophil activators can significantly reduce the expression level of FcγRIIIB, potentially related to the cleavage of GPI-linked molecules through the activation of endogenous proteases.

Functions of FcγRIII: The functions of FcγR are primarily exerted through myeloid cells and NK cells.
(1) Monocytes-macrophages: FcγRI, II, and III can all mediate ADCC in human monocytes to kill target cells such as tumors. This ADCC effect is Mg²⁺-dependent and requires the participation of adhesion molecules such as LFA-1. IFN-γ can promote the killing effect mediated by FcγRI in monocytes. Monocytes-macrophages can also exert the functions of opsonization and immune complex clearance through FcγRI, II, and III.
(2) Neutrophils: GPI-linked FcγRIIIB cannot mediate the killing of tumor cells by neutrophils. Activated neutrophils exert the functions of opsonization and immune complex clearance through FcγRI and II.
(3) NK cells: ADCC against target cells such as tumor cells is mediated by FcγRIIIA. IL-2 and IFN-γ can significantly enhance the killing activity of NK cells but do not significantly alter the expression level of FcγRIIIA.

NK cells, also known as natural killer cells, are crucial immune cells in the body that can nonspecifically and directly kill tumor cells. This natural killing activity is not restricted by MHC, does not rely on antibodies, and does not require antigen sensitization. Besides their potent killing function, NK cells also have strong immunoregulatory functions, interacting with various other immune cells in the body to regulate immune status and function of the body.

The "dynamic balance" of NK cells is mainly regulated by multiple receptor proteins on cell surface, which are divided into two categories: activating receptors (KARs) and inhibitory receptors (KIRs). The activation state, degranulation response, cytokine release, and cytolytic function of NK cells are the combined results of inhibitory and activating signals.

NKG2D (also known as CD314 and KLRK1), natural cytotoxicity receptors (NCRs), DNAM1 (also known as CD226), and CD16 are the most characteristic activating NK cell receptors involved in anti-cancer immune responses.

NK cells also express FcγRIII (CD16), a low-affinity receptor for IgG1 and IgG3, which can bind to the Fc region of tumor antigen-specific antibodies, mediating the recognition and killing of antibody-coated tumor cells by NK cells. This is also known as antibody-dependent cell-mediated cytotoxicity (ADCC).

ADCC is a process involving the activation of NK cells, the release of cytokines and cytolytic granules, as well as the induction of apoptosis in target cells. It is primarily induced and produced by the binding of low-affinity-mediated Fc receptor CD16 (FcγRIIIA or CD16a) and antibodies. Direct activation of NK cells through CD16 promotes the formation of immune synapses between NK cells and tumor cells, demonstrating cytotoxic activity against various tumor cells.

During this process, CD16 acts as the receptor that activates NK cell effector functions, inducing phosphorylation of immunoreceptor tyrosine-based activation motifs (ITAMs) through signal transduction, triggering the release of lytic granules and cytokines such as IFN-γ and TNF-α.

Moreover, when NK cells interact with target cells in the presence of specific antibodies, CD16 on NK cells undergoes downregulation of membrane expression levels while transmitting activation signals. This is a result of shedding of the extracellular fragment of CD16 during NK cell activation.

Shedding of CD16 from activated NK cells is mediated by metalloproteinase 17 (ADAM17). This demonstrates the central importance of CD16 in triggering NK cell-mediated ADCC.

### The CD16 mutant of the present invention

Compared to the point mutation strategy employed in the previously reported CD16 mutant CD16-F176V-S197P (PC) in this field (where F176V is a naturally occurring high-affinity mutant of CD16), the present invention adopts a design of inserting an amino acid P with polarity characteristics opposite to those required by the ADAM17 pocket region into one or both sides of S197 where CD16 and ADAM17 bind.

As used herein, "anti-cleavage mutant of CD16 of the present invention" and "CD16 mutant of the present invention" can be used interchangeably, referring to the CD16 mutant of the first aspect of the present invention.

Preferably, the CD16 mutant undergoes amino acid residue mutations at the following sites corresponding to wild-type CD16: insertion of one or more amino acids between the V196 and S197 sites and/or between the S197 and T198 sites of wild-type CD16; for example, the insertion of 1, 2, or 3 prolines. More preferably, the CD16 mutant further comprises the F176V mutation (CD16-F176V).

In another preferred embodiment, the mutation further comprises the deletion of one or more amino acids, or the substitution of one or more amino acids.

In another preferred embodiment, the insertion of one or more amino acids is the insertion of 1, 2, or 3 prolines.

In another preferred embodiment, the insertion of one or more amino acids comprises:
(1) insertion of 1 or 2 P between the V196 and S197 sites of CD16-F176V;
(2) insertion of 1 or 2 P between the S197 and T198 sites of CD16-F176V; or
(3) a combination of any of the above.

In another preferred embodiment, the insertion of one or more amino acids comprises:
(1) insertion of 1 P between the V196 and S197 sites of CD16-F176V;
(2) insertion of 1 P between the S197 and T198 sites of CD16-F176V; or
(3) insertion of 1 P between the V196 and S197 sites of CD16-F176V, and insertion of 1 P between the S197 and T198 sites of CD16-F176V.

Specifically, the sequences of wild-type CD16 (WT), CD16 (F176V), the previously reported CD16 mutant, and the three CD16 mutants of the present invention are as follows:
CD16-WT (SEQ ID NO. 1) 
MWQLLLPTALLLLVSAGMRTEDLPKAVVFLEPQWYRVLEKDSVTLKCQ
GAYSPEDNSTQWFHNESLISSQASSYFIDAATVDDSGEYRCQTNLSTLSDPVQL
EVHIGWLLLQAPRWVFKEEDPIHLRCHSWKNTALHKVTYLQNGKGRKYFHH
NSDFYIPKATLKDSGSYFCRGLFGSKNVSSETVNITITQGLAVSTISSFFPPGYQ
VSFCLVMVLLFAVDTGLYFSVKTNIRSSTRDWKDHKFKWRKDPQDK
CD16-F176V-S197P (PC) (SEQ ID NO. 2) 
MWQLLLPTALLLLVSAGMRTEDLPKAVVFLEPQWYRVLEKDSVTLKCQ
GAYSPEDNSTQWFHNESLISSQASSYFIDAATVDDSGEYRCQTNLSTLSDPVQL
EVHIGWLLLQAPRWVFKEEDPIHLRCHSWKNTALHKVTYLQNGKGRKYFHH
NSDFYIPKATLKDSGSYFCRGLVGSKNVSSETVNITITQGLAVPTISSFFPPGYQ
VSFCLVMVLLFAVDTGLYFSVKTNIRSSTRDWKDHKFKWRKDPQDK
 CD16-F176V -V196_S197insP **Mut1** (SEQ ID NO. 3) 
MWQLLLPTALLLLVSAGMRTEDLPKAVVFLEPQWYRVLEKDSVTLKCQ
GAYSPEDNSTQWFHNESLISSQASSYFIDAATVDDSGEYRCQTNLSTLSDPVQL
EVHIGWLLLQAPRWVFKEEDPIHLRCHSWKNTALHKVTYLQNGKGRKYFHH
NSDFYIPKATLKDSGSYFCRGLVGSKNVSSETVNITITQGLAVPSTISSFFPPGY
QVSFCLVMVLLFAVDTGLYFSVKTNIRSSTRDWKDHKFKWRKDPQDK
 CD16-F176V -S197_T198insP Mut2 (SEQ ID NO. 4) 
MWQLLLPTALLLLVSAGMRTEDLPKAVVFLEPQWYRVLEKDSVTLKCQ
GAYSPEDNSTQWFHNESLISSQASSYFIDAATVDDSGEYRCQTNLSTLSDPVQL
EVHIGWLLLQAPRWVFKEEDPIHLRCHSWKNTALHKVTYLQNGKGRKYFHH
NSDFYIPKATLKDSGSYFCRGLVGSKNVSSETVNITITQGLAVSPTISSFFPPGY
QVSFCLVMVLLFAVDTGLYFSVKTNIRSSTRDWKDHKFKWRKDPQDK
CD16-F176V -V196_S197insP-V197_T198insP Mut3 (SEQ ID NO. 5) 
MWQLLLPTALLLLVSAGMRTEDLPKAVVFLEPQWYRVLEKDSVTLKCQ
GAYSPEDNSTQWFHNESLISSQASSYFIDAATVDDSGEYRCQTNLSTLSDPVQL
EVHIGWLLLQAPRWVFKEEDPIHLRCHSWKNTALHKVTYLQNGKGRKYFHH
NSDFYIPKATLKDSGSYFCRGLVGSKNVSSETVNITITQGLAVPSPTISSFFPPGY
QVSFCLVMVLLFAVDTGLYFSVKTNIRSSTRDWKDHKFKWRKDPQDK
CD16-F176V (SEQ ID NO. 6)

MWQLLLPTALLLLVSAGMRTEDLPKAVVFLEPQWYRVLEKDSVTLK
CQGAYSPEDNSTQWFHNESLISSQASSYFIDAATVDDSGEYRCQTNLSTLS
DPVQLEVHIGWLLLQAPRWVFKEEDPIHLRCHSWKNTALHKVTYLQNGK
GRKYFHHNSDFYIPKATLKDSGSYFCRGLVGSKNVSSETVNITITQGLAVS
TISSFFPPGYQVSFCLVMVLLFAVDTGLYFSVKTNIRSSTRDWKDHKFKWR
KDPQDK

### Fusion protein

Based on the CD16 mutant of the present invention, those skilled in the art know that the CD16 mutant of the present invention can be combined with other functional portions that are not CD16 to create a fusion protein. In specific embodiments, the functional portions that are not CD16 include but are not limited to: Fc fragments, human serum albumin (HSA), anti-HSA antibodies or antibody fragments, transferrin, carboxyl-terminal peptides (CTPs) of the β-subunit of human chorionic gonadotropin, elastin-like peptides (ELPs), and antigen-binding portions. In preferred embodiments, the antigen-binding portions can be antibodies or active antibody fragments thereof, Fab molecules, scFv molecules and VHH molecules, immunoglobulin molecules, receptor protein molecules, or ligand protein molecules; the immunoglobulin molecules can be IgG molecules.

Based on routine procedures in the field, those skilled in the art know how to obtain fusion proteins comprising the CD16 mutant of the present invention. For example, the CD16 mutant of the present invention can be directly linked to other functional portions that are not CD16, or linked via a linker. The linker may be a repeated sequence of AAA or GS, including but not limited to a repeated sequences of G₃S or G₄S; for example, (G3S)₄.

A preferred class of active derivatives refers to polypeptides formed by replacing up to 3, preferably up to 2, and more preferably up to 1 amino acid with a similar or close amino acid compared to the amino acid sequence of the present invention. These conservative variant polypeptides are preferably generated by amino acid substitutions according to Table A.

**Table A**

| Initial residue | Representative substitution | Preferred substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

The present invention also provides analogs of the fusion proteins of the present invention. The differences between these analogues and the polypeptides of the present invention can be differences in amino acid sequences, differences in modification forms that do not affect the sequence, or a combination of both. Analogs also include those with residues different from natural L-amino acids (such as D-amino acids) and analogs with non-naturally occurring or synthetic amino acids (such as β, γ-amino acids). It should be understood that the polypeptides of the present invention are not limited to the representative polypeptides exemplified above.

Furthermore, modifications can also be made to the CD16 mutants or fusion proteins of the present invention. Modification (typically without altering the primary structure) forms include: chemical derivatives of polypeptides *in vivo* or *in vitro,* such as acetylation or carboxylation. Modifications also include glycosylation, such as those polypeptides produced by glycosylation modification during synthesis and processing, or further processing steps of the polypeptides. This modification can be achieved by exposing the polypeptides to enzymes that catalyze glycosylation (such as mammalian glycosylases or deglycosylases). Modification forms also include sequences with phosphorylated amino acid residues (such as phosphotyrosine, phosphoserine, phosphothreonine). Polypeptides modified to enhance their resistance to protein hydrolysis or optimize their solubility are also included.

The term "polynucleotide of the present invention" may include polynucleotides encoding the CD16 mutants or fusion proteins of the present invention, and may also include polynucleotides with additional coding and/or non-coding sequences.

The present invention also relates to variants of the aforementioned polynucleotides, which encode fragments, analogs, and derivatives of polypeptides or fusion proteins having the same amino acid sequence as those of the present invention. These nucleotide variants include substitution variants, deletion variants, and insertion variants. As known in the art, an allelic variant is an alternative form of a polynucleotide that may involve substitution, deletion, or insertion of one or more nucleotides, but does not substantially alter the function of the encoded CD16 mutant or fusion protein.

The present invention further relates to polynucleotides that hybridize with the aforementioned sequences with at least 50% identity, preferably at least 70%, and more preferably at least 80% identity between the two sequences. The present invention particularly concerns polynucleotides that can hybridize with the polynucleotides of the present invention under strict conditions (or stringent conditions). In the present invention, "strict conditions" refer to: (1) hybridization and elution at lower ionic strength and higher temperature, such as 0.2×SSC, 0.1% SDS, 60°C; or (2) hybridization in the presence of denaturants, such as 50% (v/v) formamide, 0.1% calf serum/0.1% Ficoll, 42°C, etc.; or (3) hybridization occurring only when the identity between the two sequences is at least 90%, preferably above 95%.

The CD16 mutants, fusion proteins, and polynucleotides of the present invention are preferably provided in an isolated form, and more preferably, purified to homogeneity.

The full-length sequences of the polynucleotides of the present invention can generally be obtained through PCR amplification, recombinant methods, or artificial synthesis. For PCR amplification, primers can be designed based on the related nucleotide sequences disclosed in the present invention, especially the open reading frame sequences, and commercial cDNA libraries or cDNA libraries prepared by conventional methods known to those skilled in the art can be used as templates to amplify the related sequences. When the sequence is long, it often requires two or more rounds of PCR amplification, followed by assembly of the amplified fragments in the correct order.

Once the related sequences are obtained, they can be obtained in large quantities using recombinant methods. This typically involves cloning them into vectors, transferring them into cells, and then isolating and obtaining the related sequences from the proliferated host cells using conventional methods.

In addition, related sequences can also be synthesized through artificial synthesis methods, especially when the fragment length is short. Generally, long sequence fragments can be obtained by synthesizing multiple small fragments first and then ligating them together.

Currently, DNA sequences encoding the proteins (or fragments thereof, or derivatives thereof) of the present invention can be fully obtained through chemical synthesis. The DNA sequences can then be introduced into various existing DNA molecules (or such as vectors) and cells known in the art.

The methods of amplifying DNA/RNA using PCR technology are preferred for obtaining the polynucleotides of the present invention. Especially when it is difficult to obtain full-length cDNAs from libraries, the RACE method (RACE-cDNA end rapid amplification method) can be preferably used. Primers for PCR can be appropriately selected based on the sequence information of the present invention disclosed herein and synthesized using conventional methods. The amplified DNA/RNA fragments can be separated and purified by conventional methods, such as gel electrophoresis.

### Expression vector

The present invention also relates to vectors comprising the polynucleotides of the present invention, as well as host cells genetically engineered with the vectors of the present invention or with the coding sequences of the CD16 mutant proteins or fusion proteins of the present invention, and methods for producing the polypeptides of the present invention through recombinant techniques.

Using conventional recombinant DNA technology, the polynucleotide sequences of the present invention can be utilized to express or produce recombinant fusion proteins. Generally, the following steps are involved:
(1) Transforming or transducing suitable host cells with the polynucleotide (or variant) encoding the fusion protein of the present invention, or with a recombinant expression vector comprising this polynucleotide;
(2) Culturing the host cells in an appropriate medium;
(3) Isolating and purifying the protein from the medium or cells.

In the present invention, the polynucleotide sequences encoding the fusion proteins can be inserted into a recombinant expression vector. The term "recombinant expression vector" refers to bacterial plasmids, bacteriophages, yeast plasmids, plant cell viruses, mammalian cell viruses such as adenoviruses, retroviruses, or other vectors well-known in the art. Any plasmids and vectors can be used as long as they can replicate and stabilize within the hosts. An important feature of expression vectors is that they typically contain an origin of replication, a promoter, a marker gene, and a translation control element.

In the preparation method of the CD16 mutant protein or its fusion protein of the present invention, any suitable vector can be used, which can be selected from one of pDR1, pcDNA3.1(+), pcDNA3.1/ZEO(+), and pDHFR, and the expression vector includes a fusion DNA sequence linked to appropriate transcription and translation regulatory sequences.

### Cells

In the present invention, the cells expressing the CD16 mutant can be natural killer (NK) cells, neutrophils, monocytes, T cells, macrophages, or pluripotent stem cells (such as human induced pluripotent stem cells (iPSCs) and human embryonic stem cells (ESCs), or immune cells differentiated from the pluripotent stem cells. Preferably, the cells are NK cells, which can be NK cells derived from human induced pluripotent stem cells (iPSCs) and human embryonic stem cells (ESCs); or NK cells induced and expanded from peripheral blood or umbilical cord blood; and more preferably, the cells are a population of NK cells.

Preferably, the population of NK cells can be used as a therapeutic agent. Furthermore, compared with NK cells or populations of NK cells expressing CD16 polypeptides comprising amino acid sequences shown in SEQ ID NO. 1 (wild-type) or SEQ ID NO. 6 (naturally occurring CD16 mutant CD16-F176V), the NK cells or populations of NK cells of the present invention expressing CD16 mutants comprising amino acid sequences shown in SEQ ID NO. 3, 4, or 5 have higher ADCC-mediating function, comparable to the ADCC function mediated by SEQ ID NO. 2 (CD16-F176V-S197P).

Both eukaryotic and prokaryotic host cells can be used for the expression of the CD16 mutant or its fusion protein of the present invention. Eukaryotic host cells are preferably mammalian or insect host cell culture systems, and preferably COS, CHO, NS0, sf9, and sf21 cells, etc.; prokaryotic host cells are preferably one of DH5a, BL21 (DE3), and TG1.

Methods well-known to those skilled in the art can be used to construct expression vectors comprising the DNA sequence encoding the fusion protein of the present invention and appropriate transcription/translation control signals. These methods include *in vitro* recombinant DNA technology, DNA synthesis technology, *in vivo* recombinant technology, etc. The DNA sequence can be effectively linked to an appropriate promoter in the expression vector to direct mRNA synthesis. Representative examples of these promoters include: the lac or trp promoter of *Escherichia coli*; the λ phage PL promoter; eukaryotic promoters comprising the CMV immediate-early promoter, HSV thymidine kinase promoter, early and late SV40 promoters, LTRs of retroviruses; and other known promoters that can control gene expression in prokaryotic or eukaryotic cells or viruses thereof. The expression vector also includes a ribosome binding site for translation initiation and a transcription terminator.

Furthermore, the expression vector preferably comprises one or more selective marker genes to provide phenotypic traits for selecting transformed host cells, such as dihydrofolate reductase, neomycin resistance, and green fluorescent protein (GFP) for eukaryotic cell culture, or tetracycline or ampicillin resistance for *Escherichia coli.*

Vectors comprising the appropriate DNA sequences and appropriate promoters or control sequences described above can be used to transform appropriate host cells to enable them to express the proteins.

The cells of the present invention can be host cells, such as prokaryotic cells like bacterial cells; or lower eukaryotic cells like yeast cells; or higher eukaryotic cells like mammalian cells. Representative examples include: *Escherichia coli*, Streptomyces; bacterial cells of *Salmonella typhimurium*; fungal cells such as yeast, and plant cells (such as ginseng cells).

When the polynucleotide of the present invention is expressed in higher eukaryotic cells, the insertion of an enhancer sequence into the vector will enhance transcription. Enhancers are cis-acting factors of DNA, typically about 10 to 300 base pairs, which act on promoters to enhance gene transcription. Examples include the SV40 enhancer of 100 to 270 base pairs on the late side of the replication origin, the polyomavirus enhancer on the late side of the replication origin, and the adenovirus enhancer, etc.

Those skilled in the art generally know how to select appropriate vectors, promoters, enhancers, and host cells.

Transformation of host cells with recombinant DNA can be performed using conventional techniques well-known to those skilled in the art. When the host is a prokaryote such as *Escherichia coli*, competent cells capable of absorbing DNA can be harvested after the exponential growth phase and treated with the CaCl₂ method, with the steps being well-known in the art. Another method is to use MgCl₂. If needed, transformation can also be performed using electroporation method. When the host is a eukaryote, the following DNA transfection methods can be used: calcium phosphate coprecipitation, conventional mechanical methods such as microinjection, electroporation, liposome packaging, etc.

The obtained transformants can be cultured using conventional methods to express the polypeptide encoded by the gene of the present invention. Depending on the host cell used, the medium used for culture can be selected from various conventional media. Culturing is performed under conditions suitable for the growth of the host cells. After the host cells grow to an appropriate cell density, the selected promoter is induced using an appropriate method (such as temperature shift or chemical induction), and the cells are cultured for an additional period.

The recombinant polypeptide in the above methods can be expressed within the cell, or on the cell membrane, or secreted outside the cell. If needed, the recombinant protein can be isolated and purified using various separation methods based on its physical, chemical, and other properties. These methods are well-known to those skilled in the art. Examples of these methods include, but are not limited to: conventional renaturation treatment, treatment with protein precipitating agents (salting-out methods), centrifugation, osmotic lysis, ultra-treatment, ultracentrifugation, molecular sieve chromatography (gel filtration), adsorption chromatography, ion exchange chromatography, high-performance liquid chromatography (HPLC), and other various liquid chromatography techniques, as well as combinations of these methods.

The CD16 mutant or its fusion protein disclosed in the present invention can be isolated and purified using affinity chromatography. Depending on the characteristics of the affinity column used, conventional methods such as high-salt buffer or pH adjustment can be used to elute the CD16 mutant or its fusion protein bound to the affinity column.

Using the above methods, the CD16 mutant or its fusion protein can be purified to a substantially homogeneous substance, such as a single band on SDS-PAGE electrophoresis.

### Pharmaceutical composition

In the present invention, a pharmaceutical composition comprising the aforementioned CD16 mutant or fusion protein is also provided.

The CD16 mutant or fusion protein can be formulated with pharmaceutically acceptable excipients to create a pharmaceutical preparation that exhibits therapeutic efficacy more stably. These preparations ensure the structural integrity of the amino acid core sequence of the CD16 mutant or its fusion protein of the present invention, while also protecting the multifunctional groups of the protein from degradation (including but not limited to aggregation, deamination, or oxidation). The preparations can be in various forms. Typically, for liquid preparations, they can be stably stored at 2°C-8°C for at least one year. For lyophilized preparations, they remain stable for at least six months at 30°C. Here, the preparations can be commonly used preparations in the pharmaceutical field, such as suspensions, aqueous injections, and lyophilized preparations, preferably aqueous injections or lyophilized preparations.

For the aqueous injections or lyophilized preparations of the CD16 mutant or its fusion protein of the present invention, the pharmaceutically acceptable excipients include one or a combination of surfactants, solution stabilizers, isotonic regulators, and buffers, wherein the surfactants include non-ionic surfactants such as polyoxyethylene sorbitan fatty acid esters (Tween 20 or 80); poloxamer (e.g., poloxamer 188); Triton; sodium dodecyl sulfate (SDS); sodium lauryl sulfate; tetradecyl sarcosine, linoleyl sarcosine, or octadecyl sarcosine; Pluronics; MONAQUATTM, etc., and their added amount should minimize the tendency of the protein to granulate. The solution stabilizers can be sugars, including reducing and non-reducing sugars; amino acids including monosodium glutamate or histidine; alcohols including one or a combination of triols, higher sugar alcohols, propylene glycol, and polyethylene glycol; and the added amount of solution stabilizers should be such that the final formulation is considered by those skilled in the art to remain stable for a stable period of time. The isotonic regulator can be one of sodium chloride or mannitol, and the buffer can be one of TRIS, histidine buffer, or phosphate buffer.

When administering the aforementioned CD16 mutant or its fusion protein and pharmaceutical preparation thereof to animals, including humans, the administration dosage varies based on the patient's age and weight, disease characteristics and severity, and route of administration. The total dosage should not exceed a certain range, with reference to animal experiment results and various conditions. Specifically, the intravenous injection dosage ranges from 0.1 to 3,000 mg/day.

The CD16 mutant or its fusion protein of the present invention and the pharmaceutical preparations comprising them can be used as antitumor drugs for tumor treatment. The antitumor drugs referred to in the present invention refer to drugs that inhibit and/or treat tumors, which can include delaying the development of related symptoms accompanying tumor growth and/or reducing the severity of these symptoms. They further include alleviating existing symptoms accompanying tumor growth and preventing the emergence of other symptoms, as well as reducing or preventing metastasis.

The aforementioned CD16 mutant or its fusion protein and pharmaceutical preparations thereof can also be administered in combination with other antitumor drugs for the treatment of tumors. These antitumor drugs for combination therapy include but are not limited to: 1. Cytotoxic drugs (1) Drugs that act on the chemical structure of DNA: alkylating agents such as nitrogen mustards, nitrosoureas, and methyl sulfonates; platinum compounds such as cisplatin, carboplatin, and oxaliplatin, etc.; mitomycin (MMC); (2) Drugs that affect nucleic acid synthesis: dihydrofolate reductase inhibitors such as methotrexate (MTX) and Alimta, etc.; thymidylate synthetase inhibitors such as fluorouracils (5FU, FT-207, capecitabine), etc.; purine nucleoside synthetase inhibitors such as 6-mercaptopurine (6-MP) and 6-TG, etc.; nucleotide reductase inhibitors such as hydroxyurea (HU), etc.; DNA polymerase inhibitors such as cytosine arabinoside (Ara-C) and gemcitabine (Gemz), etc.; (3) Drugs that act on nucleic acid transcription: drugs that selectively act on the DNA template, inhibit DNA-dependent RNA polymerase, and thereby inhibiting RNA synthesis, such as actinomycin D, daunorubicin, doxorubicin, epirubicin, aclacinomycin, mithramycin, etc.; (4) Drugs that mainly act on tubulin synthesis: paclitaxel, docetaxel, vinblastine, vinorelbine, podophyllotoxin derivatives, homoharringtonine; (5) Other cytotoxic drugs: asparaginase which mainly inhibits protein synthesis; 2. Hormones, anti-estrogens: tamoxifen, droloxifene, exemestane, etc.; aromatase inhibitors: aminoglutethimide, lentaron, letrozole, arimidex, etc.; anti-androgens: flutamide; RH-LH agonists/antagonists: zoladex, enantone, etc.; 3. Biological response modifiers: mainly inhibit tumors through immune function, such as interferons; interleukin-2; and thymosins; 4. Monoclonal antibodies: MabThera; Cetuximab (C225); Trastuzumab; Bevacizumab (Avastin); Yervoy (Ipilimumab); Nivolumab (OPDIVO); Pembrolizumab (Keytruda); Atezolizumab (Tecentriq); 5. Others drugs including those with currently unknown mechanisms and those that require further research; such as cell differentiation inducers like retinoids; and cell apoptosis inducers.

Preferably, when the CD16 mutant of the present invention is used in combination with antibodies that exhibit significant or observable ADCC effects (preferably antibodies such as trastuzumab, cetuximab, rituximab, etc.), it can unexpectedly significantly enhance the ADCC effects in NK cells expressing the CD16 mutant, thereby improving the antitumor effects.

### The main advantages of the present invention include:

(1) The CD16 mutant of the present invention can resist cleavage by ADAM17 and can enhance the ADCC capability of NK cells that express it.
(2) The CD16 mutant of the present invention can be used in immunotherapy to increase the anticancer activity of immune cells, especially NK cells, NK92 cells, etc.
(3) The CD16 mutant nucleotide fragment of the present invention can be inserted into specific loci (such as AAVS1) of pluripotent stem cells (iPSC, ESC, etc.) through gene editing, and differentiated immune cells represented by NK cells have stronger ability to mediate ADCC.
(4) The CD16 mutant nucleotide fragment of the present invention can be expressed on the surface of various immune cells through various gene engineering techniques (such as viral vector transfection, plasmid transfection, transposon transfection, etc.). Immune cells genetically engineered by such methods possess stronger resistance to cleavage, enhanced ADCC efficacy, and improved recognition and killing capabilities against target cells.

The present invention will be further illustrated below with reference to the specific examples. It should be understood that these examples are only to illustrate the present invention, not to limit the scope of the present invention. The conditions of the experimental methods not specifically indicated in the following examples are usually in accordance with conventional conditions as described in e.g., Sambrook et al., Molecular Cloning: A Laboratory Manual (New York: Cold Spring Harbor Laboratory Press, 1989), or according to the conditions recommended by the manufacturers. Percentages and parts are calculated by weight unless otherwise stated.

### Experimental methods

The experimental methods involved in the present invention are as follows:

### 1. Design of CD16 mutants

Three CD16 mutants were designed in this experiment. Based on CD16-F176V, a proline was inserted near the S197 site, either separately or simultaneously, and the mutants were linked to EGFP (as a reporter gene) through a P2A sequence. The corresponding fragments were inserted into the pMSCV retroviral vector, forming:
pMSCV-CD16-F176V-V196_S197insP (Mut1),
pMSCV-CD16-F176V-S197_T198insP (Mut2),
pMSCV-CD16-F176V-V196_S197insP-V197_T198insP (Mut3);

Additionally, wild-type pMSCV-CD16-WT (WT), pMSCV-CD16-WT (F176V), and a positive control pMSCV-CD16-S197P were constructed (all vectors were synthesized and extracted by Genewiz, and the sequencing results were all correct).

### 2. Cell culture

HEK293T and NK92 cells were sourced from our laboratory's cell bank, while Raji cells were purchased from ATCC. HEK293T cells were cultured in DMEM + 10% FBS medium; Raji cells were cultured in RPMI-1640 + 10% FBS medium; NK92 cell culture medium comprised α-MEM (Shanghai Peiyuan, L560KJ), 0.2mM inositol (Sigma-Aldrich, I7508), 55mM β-mercaptoethanol (MESGEN, MG1004), 20mM folic acid (Sigma-Aldrich, F8758-5G), 1000U/ml IL-2 (Novoprotein, GMP-CD66), 12.5% FBS (CAPRICORN), and 12.5% horse serum (Gibco, 26050088). Other tumor cells were expanded according to ATCC-recommended culture conditions.

### 3. Virus packaging and infection

The packaging vectors for retroviruses were BaEV-TR and pCMV-gag-pol, respectively, designed by our laboratory and synthesized and extracted by Genewiz. The virus packaging process was as follows: a) HEK-293T cells in good condition were digested and resuspended in the corresponding medium, then inoculated in 10cm culture dishes at a density of 7-8E5/ml; b) After 16h of incubation in the incubator, the cell density was observed, and plasmid transfection was initiated when the density reached about 80%; c) Two 1.5ml centrifuge tubes were prepared, each was added with 500ul of Opti-MEM^{™} I reduced serum medium (Gibco, 31985062). To one of the tubes, 7.5ug of BaEV-TR, 10ug of pCMV-gag-pol, and 20ug of the corresponding pCMV vector expressing the CD16 mutant were added; To the other tube, 40ul of PEIpro solution (polyplus, 115-010) was added; d) The solutions from the two tubes were gently mixed and allowed to stand at room temperature for 15-20min to form transfection complexes; e) The transfection complexes were gently added dropwise to the HEK-293T culture supernatant and placed in the medium for further cultivation for 4-6h; f) The medium was replaced with preheated fresh medium, and the cells were incubated in the incubator for further cultivation for 48h; g) The cell culture supernatant was collected, and the virus was concentrated using Lenti-X Concentrator (Takara, 631232); h) The virus was resuspended in 100ul of medium, and 5E5 NK92 cells were plated in a 12-well plate. 50ul of virus concentrate was added, respectively, along with a final concentration of 5ug/ml polybrene; i) The cells were centrifuged at 32°C and 800g for 1h, then incubated overnight in the incubator; j) The cells were replaced with fresh medium and cultured for an additional 3-5 days before flow cytometry analysis.

### 4. Flow cytometry analysis

Flow cytometry analysis was performed according to standard operating procedures. Taking CD16 detection as an example, the steps were as follows: a) 1E5 corresponding cells per well were collected and centrifuged at 300g for 5min at 4°C, and the supernatant was discarded; b) 200ul of precooled PBS buffer was added to each well. After gently mixing, the mixture was centrifuged at 300g for 5min at 4°C, and the supernatant was discarded; c) CD16 antibody (Biolegend, 302012) or Isotype (Biolegend, 400122) was added, and PBS was added to the remaining wells, with a volume of 100ul per well. The mixture was gently mixed and incubated at 4°C for 30min; d) the mixture was centrifuged at 300g for 5min at 4°C, and the supernatant was discarded; e) 200ul of precooled PBS buffer was added to each well. After gently mixing, the mixture was centrifuged at 300g for 5min at 4°C, and the supernatant was discarded; f) 200ul of precooled PBS buffer containing 7-AAD (BD Pharmingen^{™}, 559925) was added to each well. After gently mixing, the mixture was subjected to machine detection (Attune) and data analysis.

### 5. Resistance to ADAM17 cleavage experiment

The following four groups were set up, with Stimulation cocktail (Invitrogen, 00-4970-93) used to activate NK92 cells and ADAM17 inhibitor (Selleck, S7434, 20uM) used to specifically inhibit the activity of ADAM17. The reaction system was 100ul. After 4h, the cells were collected, and flow cytometry was used to detect CD16 expression on the surface of NK92 cells in each group.

| NK92, 1E5/well | G1 | G2 | G3 | G4 |
|---|---|---|---|---|
| Stimulation cocktail | - | - | + | + |
| ADAM17 inhibitor | - | + | - | + |

### 6. ADCC activity detection

3E4 cells of NK92-CD16-WT, NK92-CD16-PC, NK92-CD16-Mut1, NK92-CD16-Mut2, and NK92-CD16-Mut3 were taken out respectively, and were placed into one well of a 96-well plate (with 3 replicates for each group), respectively. Each type of cell was subjected to four stimulation conditions: blank, Raji (3E4), Rituximab (Selleck, A2009, 100ng/ml), and Raji + Rituximab, and the cells were placed in a culture incubator for 1h. Subsequently, Brefeldin A (Invitrogen, 00-4506-51, 1x), Monensin (Invitrogen, 00-4505-51, 1x), and Anti-human CD107a (Biolegend, 328620) were added, and the cells were further placed in the culture incubator for 3-5h, with the reaction system kept consistent among all groups during this process. Afterwards, following a standard flow cytometry staining protocol, Anti-human CD56 (Biolegend, 362508) and 7-AAD were added for staining. The cells were then subjected to machine detection and data analysis.

### Example 1 Design of CD16 mutants

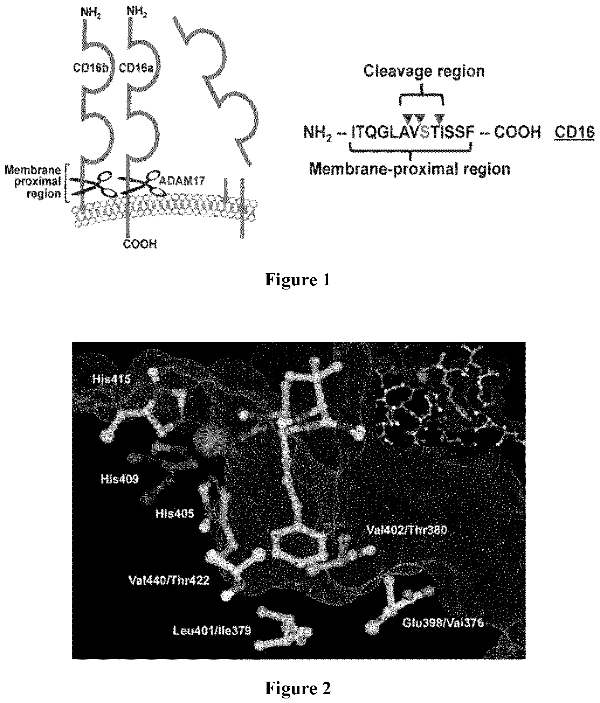

Considering that CD16 shedding negatively regulates the ADCC function of cells expressing CD16, such as NK cells, thereby weakening the efficacy of cellular and antibody drugs during long-term treatment, to overcome the effect of this negative regulatory signal, the applicant analyzed the molecular structure of ADAM17 (Figure 2) and the molecular mechanism by which ADAM17 cleaves peptide chains. Based on the binding mode between CD16 and the enzymatic active pocket of ADAM17, three conformational mutants of CD16 were designed. It was anticipated that these three mutants would not be cleaved by ADAM17.

Through experiments, the applicant has confirmed that these three CD16 mutants can indeed resist cleavage by ADAM17 and enhance the ADCC capacity of the NK92 cell line expressing them.

Compared to the point mutation strategy employed in the previously reported CD16 mutant CD16-F176V-S197P (positive control, PC) (where F176V is a naturally occurring high-affinity mutant of CD16), the three mutants designed by the applicant are:
CD16-F176V-V196_S197insP (Mut1),
CD16-F176V-S197_T198insP (Mut2), and
CD16-F176V-V196_S197insP-V197_T198insP (Mut3),
which adopt a design of inserting an amino acid P with polarity characteristics opposite to those required by the ADAM17 pocket region into one or both sides of S197 where CD16 and ADAM17 bind.

Experimental results have proven that this approach indeed functions as intended.

### Example 2 CD16 mutants can achieve resistance to ADAM17 cleavage

Wild-type CD16 (WT), the previously reported positive control CD16 mutant (PC), and the three CD16 mutants designed by the applicant were overexpressed in the NK92 cell line using a γ-retroviral vector.

To verify whether ADAM17 cleaves these mutants, four experimental conditions G1-G4 were set up (Table 1). Among them, the PMA/Ionomycin mixture was used to activate NK92 cells, and TAPI-1 was a small molecule inhibitor of ADAM17.

**Table 1 ADAM17 cleavage experiment grouping design**

| | G1 | G2 | G3 | G4 |
|---|---|---|---|---|
| PMA/Ionomycin | - | - | + | + |
| TAPI-1 (20µM) | - | + | - | + |

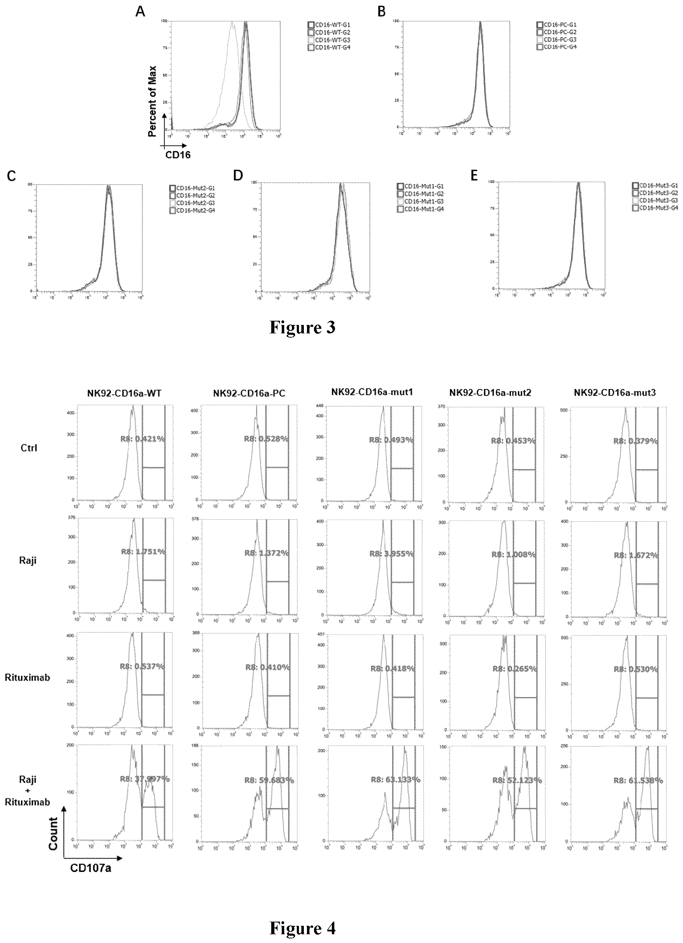

It can be seen (Figure 3, A) that for wild-type CD16 (WT), NK92 cells expressed higher levels of CD16 in the inactive state (G1, G2); upon the action of PMA/Ionomycin, NK cells were activated, and the expression levels of CD16 were significantly reduced (G3); after adding TAPI-1, the activity of ADAM17 was inhibited, and the expression of CD16 returned to normal levels (G4).

The above experimental results have shown that wild-type CD16 (WT) can be specifically cleaved by ADAM17 in activated NK92 cells. Compared to wild-type CD16 (WT), the positive control (PC) and the three mutants of the invention (Mut1, Mut2, Mut3) exhibit good resistance to ADAM17 cleavage (Figure 3, BE).

### Example 3 CD16 mutants mediate stronger NK92 cell activation

To further verify whether the CD16 mutants of the invention have the function to mediate ADCC, the expression of CD107a (a marker of NK92 activation) was detected in each group of cells under different stimulation conditions.

It can be seen (Figure 4) that in the presence of only Raji cells or Rituximab (a monoclonal antibody targeting CD20), the NK92 cells in each group could not be effectively activated; however, when Raji cells and Rituximab were present simultaneously, the NK92 cells were activated to express CD107a. Like the positive control (PC), the three CD16 mutants of the invention can resist cleavage by ADAM17, thereby better mediating ADCC and more strongly activating NK92 cells.

The above experimental results have shown that the three CD16 mutants of the invention can all mediate stronger ADCC effects.

### Example 4 CD16 mutants mediate stronger ADCC effects

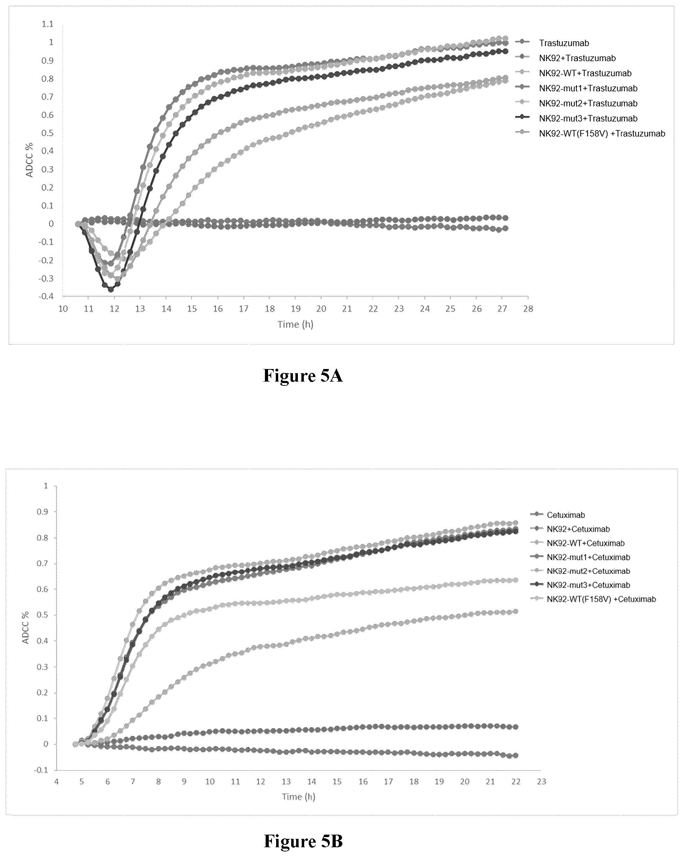

To demonstrate that NK92 cells expressing CD16 mutants have stronger capability to mediate ADCC, we performed ADCC detection on SKOV3 (90% HER2 expression) and A549 (100% EGFR expression) using Trastuzumab and Cetuximab, respectively. The results have shown that compared to NK92 expressing wild-type CD16 (including CD16WT and CD16WT (F176V)), NK92 expressing CD16 mutants can mediate stronger ADCC effects (Figure 5A, 5B). ADCC was calculated as: ADCC% = (Experimental cytotoxicity - Ab-independent cytotoxicity) / Maximum cytotoxicity * 100%.

### Example 5 iNKs expressing CD16-mut3 exhibit resistance to cleavage and stronger ADCC capability

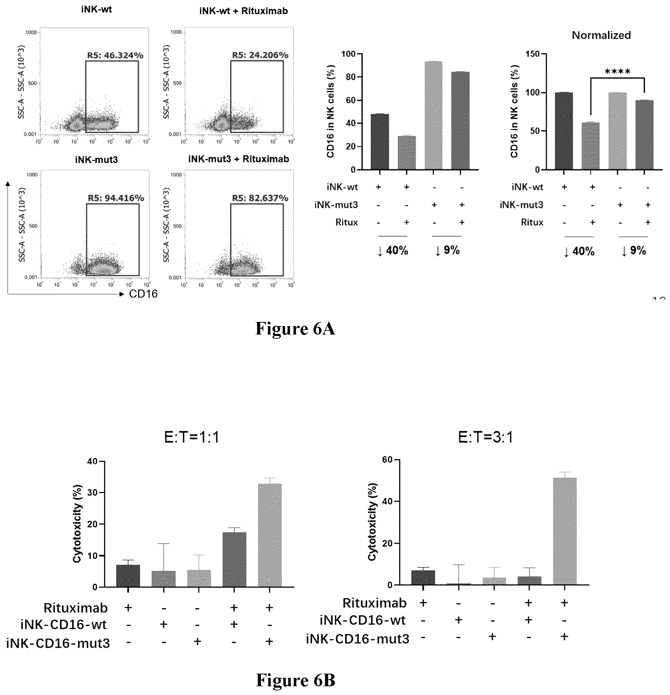

To further verify the function of CD16 mutants in NK (iNK) cells derived from induced pluripotent stem cells (iPSCs), CD16-mut3 was knocked into the iPSC genome using CRISPR/Cas9, and iPSC monoclones expressing CD16-mut3 were screened and expanded for iNK differentiation, ultimately obtaining iNKs with over 90% CD16-mut3 expression (iNK-mut3). For unedited iPSCs, after differentiation, they expressed approximately 45% of wild-type CD16 (iNK-wt). After adding Raji cells and Rituximab to both groups of cells, respectively, iNK cells were activated and produced an ADCC effect. The expression of wild-type CD16 decreased by 40%, while the expression of CD16-mut3 decreased by only 9%, showing significant resistance to cleavage (Figure 6A).

At the same time, the ADCC effects of iNK-wt and iNK-mut3 on Raji cells were tested, and it was observed that iNK-mut3 exhibited stronger ADCC effects (Figure 6B).

### Example 6 iNKs expressing CD16-mut3 exhibit strong ADCC effects on multiple tumor cells

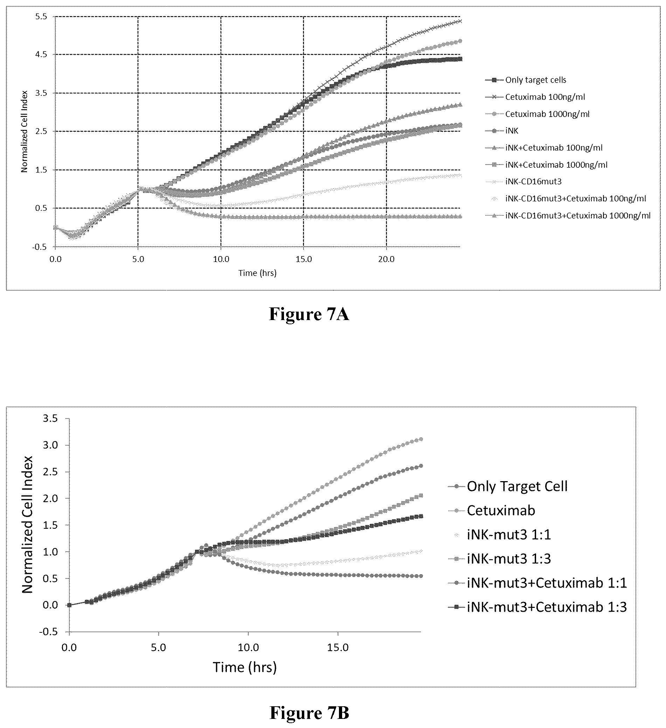

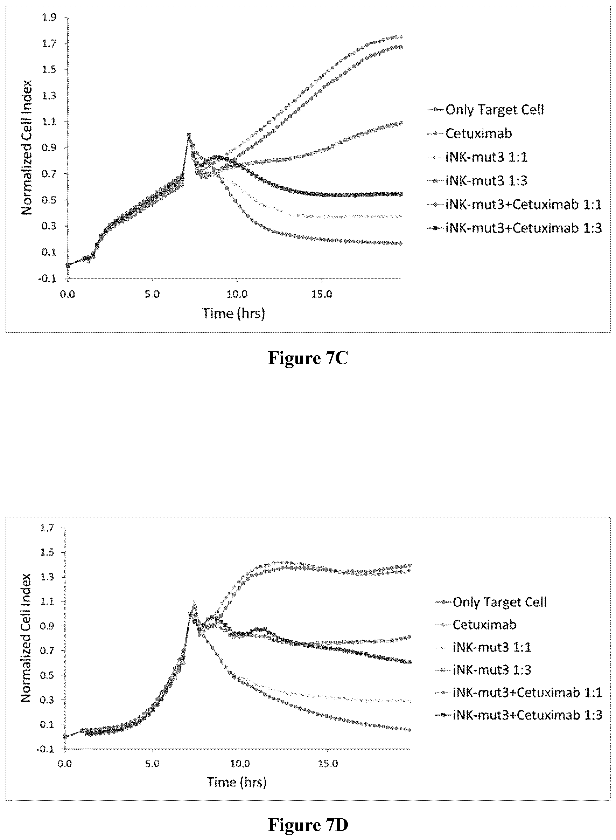

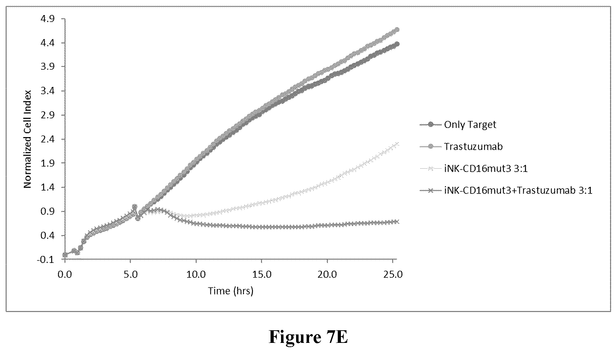

Subsequently, ADCC detection was further conducted on A549 (lung cancer), Cal27 (head and neck cancer), Cal33 (head and neck cancer), FADU (head and neck cancer), and HCC-1954 (breast cancer) cells, etc. At an E:T ratio of 3:1, compared to iNKs, iNKs expressing CD16-mut3 exhibited stronger ADCC killing ability (Figure 7A). At the same time, iNK-mut3 (iNK-vCD16) also showed strong ADCC effects on Cal27 (Figure 7B), Cal33 (Figure 7C), FADU (Figure 7D), and SKOV3 (Figure 7E) cells, etc.

The above results have shown that when used in combination with antibodies, the iNKs obtained by editing CD16-mut3 into iPSCs and then differentiating, have stronger ADCC function and potential clinical development and application prospects.

All literatures mentioned in the present invention are incorporated herein by reference, as though each one is individually incorporated by reference. In addition, it should be understood that, after reading the above teachings of the present invention, those skilled in the art can make various changes or modifications to the present invention, these equivalents also fall within the scope as defined in the appended claims of the present application.

## Claims

1. A CD16 mutant, wherein the CD16 mutant undergoes amino acid residue mutations at the following sites corresponding to wild-type CD16: insertion of one or more amino acids between the V196 and S197 sites and/or between the S197 and T198 sites of wild-type CD16; wherein the insertion of one or more amino acids comprises the insertion of one or more prolines.

2. The CD16 mutant of claim 1, wherein the CD16 mutant further comprises a mutation where the F at position 176 of wild-type CD16 is replaced with V (F176V).

3. The CD16 mutant of claim 1, wherein the insertion of one or more amino acids comprises:
(1) insertion of 1 P between the V196 and S197 sites of CD16-F176V;
(2) insertion of 1 P between the S197 and T198 sites of CD16-F176V; or
(3) insertion of 1 P between the V196 and S197 sites of CD16-F176V, and insertion of 1 P between the S197 and T198 sites of CD16-F176V.

4. A fusion protein comprising the CD16 mutant of claim 1 and a functional portion that is not a CD16 mutant.

5. The fusion protein of claim 4, wherein the functional portion that is not a CD16 is selected from the group consisting of:
Fc fragments, including but not limited to: Fc fragments of human IgG1, IgG2, IgG3, or IgG4, and Fc fragment mutants thereof with homology of 90% or higher;
human serum albumin (HSA);
6His tag.

6. A polynucleotide encoding the CD16 mutant of claim 1 or the fusion protein of claim 4.

7. An expression vector comprising the polynucleotide of claim 6.

8. A cell comprising the expression vector of claim 7, or the genome of the cell is integrated with the polynucleotide of claim 6.

9. The cell of claim 8, wherein the cell is a natural killer (NK) cell, neutrophil, monocyte, T cell, macrophage, pluripotent stem cell, or a cell differentiated from the pluripotent stem cell.

10. The cell of claim 9, wherein the pluripotent stem cell comprises a human induced pluripotent stem cell (iPSC) or human embryonic stem cell (ESC), or the cell differentiated from the pluripotent stem cell is an immune cell.

11. The cell of claim 9, wherein the NK cell is a NK cell derived from a human induced pluripotent stem cell (iPSC) and human embryonic stem cell (ESC), or a NK cell induced or expanded from peripheral blood or umbilical cord blood.

12. A pharmaceutical composition comprising the CD16 mutant of claim 1, the fusion protein of claim 4, or the cell of claim 8, and a pharmaceutically acceptable carrier.

13. The pharmaceutical composition of claim 4, wherein the pharmaceutical composition further comprises other drugs used for treating tumors; the other drugs used for treating tumors (i) specifically recognize tumor antigens; or (ii) comprise antibodies or antibody fragments that specifically recognize tumor antigens; or (iii) specifically recognize viral target proteins.

14. An *in vitro* method for enhancing ADCC activity in mammalian cells, which comprises introducing the polynucleotide of claim 6 into the cells.

15. Use of an active ingredient combination, wherein the active ingredient combination comprises a first active ingredient: the CD16 mutant of claim 1, the fusion protein of claim 4, or the cell of claim 8; and a second active ingredient: an antibody or antibody fragment that specifically recognizes tumor antigens, and the combination is used for: enhancing ADCC activity in mammalian cells; and/or preparing a pharmaceutical composition or kit for treating tumors.
